# EUROPEAN PATENT SPECIFICATION

(11) **EP 0 608 041 B1**
(45) Date of publication and mention of the grant of the patent: **26.01.2000**
(21) Application number: 94200798.0
(22) Date of filing: 17.10.1986
(51) Int. Cl.: C07D 207/404, C10M 133/56, C10L 1/22, C10M 133/16, C10N 30/04, C10N 40/25

(54) **Compositions, concentrates, lubricant compositions, fuel composition and methods for improving fuel economy of internal combustion engines**
Zusammensetzungen, Konzentrate, Schmiermittelzusammensetzung, Brennstoffzusammensetzung, und Verbesserungsverfahren der Brennstoffersparnis von Verbrennungsmotoren
Compositions, concentrés, composition de lubrifiant, composition de carburant et méthode pour améliorer les économies de carburant dans des moteurs à combustion interne

(30) Priority: 25.10.1985 US 791260
(43) Date of publication of application: 27.07.1994
(62) Divisional of application: 91110118.6
(73) Proprietor: THE LUBRIZOL CORPORATION, Wickliffe, Ohio 44092-2298 (US)
(72) Inventor: Walsh, Reed H., Mentor, Ohio 44060 (US)
(74) Representative: Mallalieu, Catherine Louise

(56) References cited:
- EP-A- 0 008 953
- EP-A- 0 020 037
- US-A- 3 068 082

## Description

### BACKGROUND OF THE INVENTION

This invention relates to compositions useful as lubricant and fuel additives and methods for improving the operation of internal combustion engines, specifically by reducing the amount of fuel consumed by such engines. More particularly, the invention comprises lubricating compositions which may be used in such engines to decrease fuel consumption, and a method of using such lubricating compositions to accomplish this purpose. This invention also relates to a method for preparing these compositions. Additionally, this invention relates to concentrates and fuel composition comprising these compositions.

Efforts to reduce the amount of fuel consumed by internal combustion engines such as automobile engines have increased in recent years as a result of the petroleum shortage, the increased cost of petroleum products, and the desire for conservation of natural resources such as petroleum. It is recognized that a situation under which fuel consumption is minimized is desirable, both because of the conservation factor and because such a situation is economical for the user of the engine.

Many of the proposed solutions to the fuel conservation problem have been mechanical as, for example, adjusting the engine for a leaner burn or simply building smaller cars and smaller engines. Other efforts have related to developing lubricants that reduce the overall friction of the engine thereby reducing energy requirements. Some synthetic lubricants have been developed and compounded for use in the automobile engine to reduce fuel consumption. A considerable amount of effort has been expended toward developing additives for use in mineral lubricating oils and greases to reduce the friction properties of the oils and greases.

The following publications are exemplary of art in this area:
U.S. Patent 3,796,663 describes N-hydroxy hydrocarbyl-substituted cyclic imides of C₄-C₅dicarboxylic acids, e.g., hydrocarbyl-substituted succinyl and glutaric hydroximides, as rust inhibition, wear reduction and frictional control additives for lubricating oils.

U.S. Patent 4,104,182 describes a lubricating oil composition comprising a hydrocarbyl oil of lubricating viscosity, a metal-containing additive characterized by promoting the formation of hard deposits in an internal combustion engine, and a hydrocarbon-substituted succinimide represented by the formula: 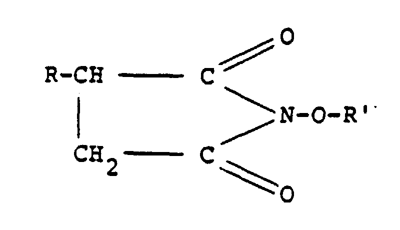 in which R is an aliphatic hydrocarbon radical having from about 1 to 50 carbon atoms and R' is a hydrocarbon radical having from 3 to 20 carbon atoms, and a method for lubricating an internal combustion engine.

U.S. Patent 4,325,827 describes a fuel efficient motor oil which contains a friction-reducing amount of an N-hydroxymethyl C₁₂₋₃₆ aliphatic hydrocarbyl succinimide. This patent also describes these additives as being used in liquid hydrocarobn engine fuel.

European Patent Application 20,037 describes oil soluble C₁₂₋₃₆ aliphatic hydrocarbyl succinimide or succinamide which provides a friction reducing effect where it is incorporated in a lubricating oil.

Furthermore, EP-A-0 008 953 describes fuel compositions comprising an alkenyl succinimide as a detergent. The alkenyl succinimide that may be used for this purpose is prepared by reacting an alkenylsuccinic acid or anhydride, wherein the alkenyl substituent is derived from a mixture of C₁₆-C₂₈ olefins, with a polyalkylene polyamine of formula

NH₂ - (RNH)ₙR-NH₂

wherein R is alkylene having from 1 to 5 carbon atoms and n is from 0 to 10. This document thus suggests the preparation of alkenyl succinimides from a polyamine where n is 0 and R is methylene, i.e. methylene diamine, but such a polyamine is not known to exist.

### SUMMARY OF THE INVENTION

In its broadest sense, the present invention provides a substituted succinimide derivative represented by the formula: 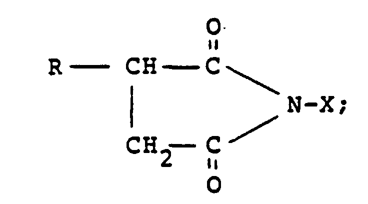 wherein X is 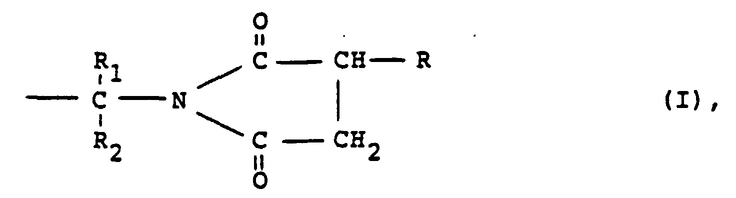 wherein R is a hydrocarbon-based group containing from about 8 up to about 35 carbon atoms, and R₁ and R₂ are each independently hydrogen or an alkyl group containing up to 8 carbon atoms. Lubricating oils containing the compositions of the invention are effective in reducing the amount of fuel consumed by internal combustion engines. The invention also relates to a method of reducing the amount of fuel consumed by an internal combustion engine. In addition, lubricating oils containing the compositions of this invention are effective deposit softeners in internal combustion engines. This invention also relates to methods for preparing these substituted succinimide derivatives which are discussed hereinafter.

### DETAILED DESCRIPTION OF THE INVENTION

In the substituted succinimide derivatives of this invention, R is a hydrocarbon-based group containing from about 8 up to about 35 carbon atoms. As used herein, the term "hydrocarbon-based group" denotes a radical having a carbon atom directly attached to the remainder of the molecule and having predominantly hydrocarbon character within the context of this invention. Such groups include the following:
(1) Hydrocarbon groups; that is, aliphatic (e.g., alkyl or alkenyl), alicyclic (e.g., cycloalkyl or cycloalkenyl), aromatic, aliphatic- and alicyclic-substituted aromatic, aromatic-substituted aliphatic and alicyclic radicals, and the like, as well as cyclic radicals wherein the ring is completed through another portion of the molecule (that is, any two indicated substituents may together form an alicyclic radical). Such groups are known to those skilled in the art.
(2) Substituted hydrocarbon groups; that is, radicals containing nonhydrocarbon substituents which, in the context of this invention, do not alter the predominantly hydrocarbon character of the radical. Those skilled in the art will be aware of suitable substituents; examples are halo, alkoxy, hydroxy, alkylthio, carbalkoxy, nitro and carboxyl.
(3) Hetero groups; that is, radicals which, while predominantly hydrocarbon in character within the context of this invention, contain atoms other than carbon present in a chain or ring otherwise composed of carbon atoms.
Suitable hetero atoms will be apparent to those skilled in the art and include, for example, nitrogen, oxygen and sulfur.

In general, no more than about three substituents or hetero atoms, and preferably no more than one, will be present for each 10 carbon atoms in the hydrocarbon-based group.

Terms such as "alkyl hydrocarbon-based group," "aliphatic hydrocarbon-based group," "aryl hydrocarbon-based group" and the like have meanings analogous to the above with respect to alkyl, aliphatic and aryl groups and the like.

Preferably, the hydrocarbon-based groups in the compositions of this invention are free from acetylenic unsaturation.

As used in the present specification and claims, the term "lower," when used in conjunction with terminology designating a chemical group such as alkyl, alkenyl, alkylene and the like, is intended to describe such groups having a total carbon atom content of up to and including 7. For example, "lower alkyl" includes all straight and branched chain alkyl groups of up to and including 7 carbon atoms.

Typically the substituent groups are aliphatic hydrocarbon-based groups containing from about 8 up to about 35 carbon atoms, preferably from about 10 up to about 30 carbon atoms and more preferably from about 12 up to about 28 carbon atoms. The R group may be a branched chain or straight chain configuration; however, it is preferred that at least 8 carbon atoms are in a straight chain configuration and, more preferably, is substantially straight chain configuration. Furthermore, the R group is preferably alkyl or alkenyl.

The term "substantially straight-chain" means that the group contains no more than about 2 methyl groups.

Generally R₁ and R₂ are each independently hydrogen or an alkyl group containing up to 8 carbon atoms, preferably up to about 3 carbon atoms. Usually, at least one of R₁ and R₂ is hydrogen and, preferably, both R₁ and R₂ are hydrogen.

The substituted succinimide derivatives of the instant invention in which X is represented by the formula: 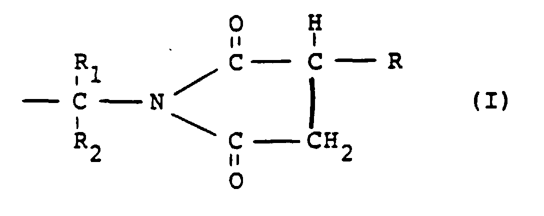 wherein R, R₁ and R₂ are defined hereinabove can be prepared by reacting in the presence of a catalytic amount of base:
A) one or more substituted succinimides represented by the formula: 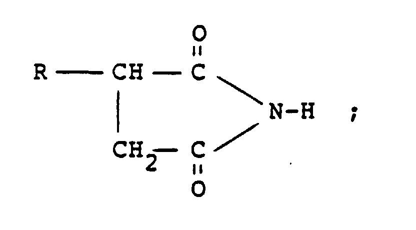 wherein R is a hydrocarbon-based group containing from about 8 up to about 35 carbon atoms; with
B) one or more carbonyl compounds represented by the formula: 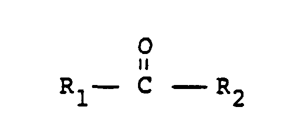 wherein R₁ and R₂ are each independently hydrogen or an alkyl radical containing up to 8 carbon atoms.

The substituted succinimide useful for preparing the substituted succinimide derivatives of the present invention are typically prepared by reacting a substituted succinic acid or derivative thereof, with ammonia to form the imide. The substituted succinic acids and their derivatives (e.g., anhydrides, acid halides, esters) of this type may be conveniently prepared by the reaction of maleic or fumaric acid or a derivative thereof, preferably maleic anhydride, with one or more olefins containing from about 8 to about 35 carbon atoms.

The substituted succinimides and the substituted succinic acid or derivatives thereof useful for the purposes of this invention are well known to those of ordinary skill in the art. Some of these are described in U.S. Patents 4,325,827; 4,324,872; 4,158,664; 4,000,163; 3,819,660; 3,796,663; 3,412,111; 3,382,172; and 2,411,215.

The base catalyst may be inorganic oxides and salts such as sodium hydroxide, calcium oxide, calcium hydroxide, sodium sulfide, sodium carbonate and sodium bicarbonate Other base catalysts include sodium acetate, sodium propronate and amines. The amines include primary, secondary and tertiary hydrocarbyl amines wherein the hydrocarbyl radicals are alkyl, aryl, aralkyl, alkaryl or the like and contain about 1-20 carbon atoms. Suitable amines include aniline, benzylamine, dibenzylamine, dodecylamine, naphthylamine, tallow amines, N-ethyldipropylamine, N-phenylbenzylamine, N,N-diethylbutylamine, m-toluidine and 2,3-xylidine. Also useful are heterocyclic amines such as pyrrolidine, N-methylpyrrolidine, piperidine, pyridine and quinoline.

The preferred basic catalyst is sodium hydroxide.

The amount of catalytic material used is generally about 0.05 to 2.0 percent of the weight of the reaction mixtures. In the case of the preferred sodium hydroxide catalyst, about 0.005-0.5 mole per mole of substituted succinimide is preferred, and about 0.001-0.1 mole is especially desirable.

The carbonyl compounds of this invention are the aldehydes and ketones corresponding to the formula: 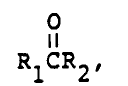 wherein R₁ and R₂ are as previously defined. Examples of aldehydes which are within the scope of this invention include formaldehyde, acetalaldehyde, propionaldehyde, n-butyraldehyde, isobutyraldehyde, n-valeraldehyde, isovaleraldehyde, alpha-methylbutyraldehyde, n-caprioicaldehyde, isocaprioicaldehyde, 2-ethylbutyraldehyde, methyl-n-propylacetaldehyde, ethylisobutyraldehyde, n-heptaldehyde, ethylisopropylacetaldehyde, 3,3-dimethylpentanal, 5-methylhexanal, caprylaldehyde, 2-ethylhexanal-1, capraldehyde, di-n-propylacetaldehyde, and the like. Mixtures of two or more such aldehydes can be used.

The preferred aldehydes are formaldehyde or functional equivalents thereof. Such functional equivalents are materials (e.g., solutions, polymers, hydrates, etc.) which react as aldehydes under the conditions of the reaction and include paraformaldehyde, formalin and methylal. Examples of ketones which are in the scope of this invention include acetone, methylethyl ketone, 2-pentanone, 2-methyl-3-butanone, 3-hexanone, 4-methyl-2-pentanone, cyclobutylmethylketone, 1-methoxy-2-pentanone, 2-heptanone, 4-heptanone, 2,2-dimethyl-3-pentanone, 2,4-dimethyl-3-pentanone, and the like. Mixtures of two or more such ketones can be used. Also, mixtures of one or more aldehydes and one or more ketones can be used. Preferably, the carbonyl compound is an aldehyde and, more preferably, formaldehyde or functional equivalent thereof.

Usually, there is at least about one mole of carbonyl compound for every two moles of succinimide, preferably, about one mole of carbonyl compound for every two moles of succinimide.

The reaction process for the preparation of the compositions of this invention involving the use of a carbonyl compound is usually carried out for a period long enough for the condensation to be substantially complete. The reaction is considered complete when all the water of reaction is formed (one mole of water for each mole of carbonyl compound).

For practical purposes, one of ordinary skill in the art could determine the extent of water formation by standard techniques such as distillation, separation, and the like. Preferably, the water of reaction is removed continuously as it is formed. For purposes of this invention, the reaction is considered complete when about 90% of the theoretical amount of water to be formed (one mole of water for each mole of carbonyl compound) has been removed by such standard techniques. The reaction period can be about 0.5 to 72 hours, but is usually 0.5 to 24 hours at a temperature of from about 140°C up to just below the decomposition temperature of any component of the reaction mixture, usually from about 180° up to 260°C. Should any of the ingredients have a boiling point below that of the desired reaction temperature, the reaction may be conveniently carried out at superatmospheric pressures.

The reactions of this invention may be carried out in the presence of a substantially inert liquid solvent/diluent medium. This solvent/diluent medium desirably serves to maintain contact of the reactants and facilitates control of the reaction temperature. Examples of suitable solvent/diluent media include aliphatic and aromatic hydrocarbons as benzene, toluene, naphtha, mineral oil, hexane; chlorinated hydrocarbons as dichlorobenzene, and heptylchloride; ethers as methyl n-amylether, n-butylether.

As used in the specification and the appended claims, the term "substantially inert" when used to refer to solvents/diluents, and the like, is intended to mean that the solvent/diluent, etc., is sufficiently inert to chemical or physical change under the conditions in which it is used so as not to materially interfere in an adverse manner with the preparation, storage, blending and/or functioning of the compositions, additive, compound, etc., of this invention in the context of its intended use. For example, small amounts of a solvent/diluent, etc., can undergo minimal reaction or degradation without preventing the making and using of the invention as described herein. In other words, such reaction or degradation, while technically discernible, would not be sufficient to deter the practical worker of ordinary skill in the art from making and using the invention for its intended purposes. "Substantially inert" as used herein is, thus, readily understood and appreciated by those of ordinary skill in the art.

As used in the specification and the appended claims, the term "solvent/diluent medium" is intended to include those solvent/diluent media in which independently each of the reactants are soluble or stably dispersible. The term "stably dispersible" as used in the specification and the appended claims is intended to mean a composition (e.g., a single compound, a mixture of two or more compounds, etc.) is capable of being dispersed in a given medium to an extent which allows it to function in its intended manner. Thus, for example, where a composition is prepared by a reaction in an oil, it is sufficient that the reactants be capable of being suspended in the oil in a manner sufficient to allow the reaction to occur and the formation of the composition. Thus, the term "solvent/diluent medium" is understood and can be used in a conventional manner by those of ordinary skill in the art.

The compositions of this invention may be used as a lubricant additive. However, the compositions sometimes may be accompanied by the formation of by-products and/or excess solvent/diluent medium which may lessen its commercial appeal. Accordingly, these undesirable by-product and/or excess of undesired solvent/diluent medium can be separated from the compositions of this invention by techniques known in the art; e.g., filtration, evaporation (e.g., stripping), etc., to obtain a more desirable product. Alternatively, if the solvent/diluent medium is, for example, a lubricant base suitable for use in the lubricating compositions of this invention, the product can be left in the solvent/diluent medium and used to form the lubricating compositions as described below.

This invention is exemplified in the following example. Of course, this example is not intended as limiting this invention as modification of the example by ordinary expedient will be readily apparent to those of ordinary skill in the art.

In the example, unless otherwise stated, all parts are parts by weight and all percentages are derived from parts by weight.

### Example

Charged to a reaction vessel are 198 parts (2.02 moles) of maleic anhydride and 500 parts (1.36 moles) of a commercial mixture of C₁₈₋₂₄ olefins available from Ethyl Corporation wherein these olefins are typically 10% C₁₈, 45% C₂₀, 25% C₂₂ and 15% C₂₄ and are comprised predominantly of substantially straight chain alpha, 1,1-disubstituted and 1,2-disubstituted olefins. This reaction mixture is heated to 200°C and held at 200-220°C for 10 hours. Unreacted starting materials are removed by vacuum distillation to 5 mm Hg. at 200°C. The reaction mixture is filtered to yield the desired C₁₈₋₂₄ substituted succinic anhydride having an acid number of 290.

A reaction vessel containing 5424 parts (12 moles) of the C₁₈₋₂₄ substituted succinic anhydride is heated to 120°C. Gaseous ammonia, 267 parts (15.7 moles), is slowly bubbled below the surface over 6.75 hours during which the temperature of the reaction mixture is increased to 240°C. Water of reaction is removed continuously during the NH₃ addition. The reaction mixture is stripped to 190°C. The residue is the C₁₈₋₂₄ substituted succinimide having an acid number to phenolphthalein of 69 and a per cent nitrogen of 2.91.

A mixture of 902 parts (2 moles) of the C₁₈₋₂₄ substituted succinimide, 100 parts xylene, 8 parts (0.1 moles) of a 50% aqueous sodium hydroxide solution and 33 parts (1.1 moles) paraformaldehyde is heated under a nitrogen blanket to 165°C and water is removed by azeotropic distillation. The remaining volatiles are removed by vacuum distillation. The reaction mixture is filtered through a filter aid material to yield the desired product having a 2.83% nitrogen content and an acid number to phenolphthalein of 52.

As previously indicated, the compositions of this invention are also useful as additives for lubricants, in which they can function primarily as friction modifiers and/or deposit softeners. They can be employed in a variety of lubricants based on diverse oils of lubricating viscosity, including natural and synthetic lubricating oils and mixtures thereof. These lubricants include crankcase lubricating oils for spark-ignited and compression-ignited internal combustion engines, including automobile and truck engines, two-cycle engines, aviation piston engines, marine and railroad diesel engines, and the like. They can also be used in gas engines, stationary power engines and turbines and the like. Automatic transmission fluids, transaxle lubricants, gear lubricants, metal-working lubricants, hydraulic fluids and other lubricating oil and grease compositions can also benefit from the incorporation therein of the compositions of the present invention.

Natural oils include animal oils and vegetable oils (e.g., castor, lard oil) liquid petroleum oils and hydrorefined, solvent-treated or acid-treated mineral lubricating oils of the paraffinic, naphthenic and mixed paraffinic-naphthenic types. Oils of lubricating viscosity derived from coal or shale are also useful base oils.

Synthetic lubricating oils include hydrocarbon oils and halo-substituted hydrocarbon oils such as polymerized and interpolymerized olefins (e.g., polybutylenes, polypropylenes, propylene-isobutylene copolymers, chlorinated polybutylenes, poly(1-hexenes), poly(1-octenes), poly (1-decenes)); alkylbenzenes (e.g., dodecylbenzenes, tetradecylbenzenes, dinonylbenzenes, di(2-ethylhexyl)benzenes); polyphenyls (e.g., biphenyls, terphenyls, alkylated polyphenols); and alkylated diphenyl ethers and alkylated diphenyl sulfides and the derivatives, analogs and homologs thereof.

Alkylene oxide polymers and interpolymers and derivatives thereof where the terminal hydroxyl groups have been modified by esterification, etherification, etc., constitute another class of known synthetic lubricating oils. These are exemplified by polyoxyalkylene polymers prepared by polymerization of ethylene oxide or propylene oxide, the alkyl and aryl ethers of these polyoxyalkylene polymers (e.g., methyl-polyisopropylene glycol ether having an average molecular weight of 1000, diphenyl ether of poly-ethylene glycol having a molecular weight of 500-1000, diethyl ether of polypropylene glycol having a molecular weight of 1000-1500); and mono- and polycarboxylic esters thereof, for example, the acetic acid esters, mixed C₃-C₈ fatty acid esters and C₁₃ Oxo acid diester of tetraethylene glycol.

Another suitable class of synthetic lubricating oils comprises the esters of dicarboxylic acids (e.g., phthalic acid, succinic acid, alkyl succinic acids and alkenyl succinic acids, maleic acid, azelaic acid, suberic acid, sebacic acid, fumaric acid, adipic acid, linoleic acid dimer, malonic acid, alkylmalonic acids, alkenyl malonic acids) with a variety of alcohols (e.g., butyl alcohol, hexyl alcohol, dodecyl alcohol, 2-ethylhexyl alcohol, ethylene glycol, diethylene glycol monoether, propylene glycol). Specific examples of these esters include dibutyl adipate, di(2-ethylhexyl) sebacate, di-n-hexyl fumarate, dioctyl sebacate, diisooctyl azelate, diisodecyl azelate, dioctyl phthalate, didecyl phthalate, dieicosyl sebacate, the 2-ethylhexyl diester of linoleic acid dimer, and the complex ester formed by reacting one mole of sebacic acid with two moles of tetraethylene glycol and two moles of 2-ethylhexanoic acid.

Esters useful as synthetic oils also include those made from C₅ to C₁₂ monocarboxylic acids and polyols and polyol ethers such as neopentyl glycol, trimethylolpropane, pentaerythritol, dipentaerythritol and tripentaerythritol, etc.

Silicon-based oils such as the polyalkyl-, polyaryl-, polyalkoxy-, or polyaryloxysiloxane oils and silicate oils comprise another useful class of synthetic lubricants; they include tetraethyl silicate, tetraisopropyl silicate, tetra-(2-ethylhexyl) silicate, tetra-(4-methyl-2-ethylhexyl) silicate, tetra-(p-tert-butylphenyl) silicate, hexa-(4-methyl-2-pentoxy)disiloxane, poly(methyl)siloxanes and poly(methylphenyl) siloxanes. Other synthetic lubricating oils include liquid esters of phosphorus-containing acids (e.g., tricresyl phosphate, trioctyl phosphate, diethyl ester of decylphosphonic acid) and polymeric tetrahydrofurans.

Unrefined, refined and rerefined oils can be used in the lubricants of the present invention. Unrefined oils are those obtained directly from a natural or synthetic source without further purification treatment. For example, a shale oil obtained directly from retorting operations, a petroleum oil obtained directly from distillation or ester oil obtained directly from an esterification process and used without further treatment would be an unrefined oil. Refined oils are similar to the unrefined oils except they have been further treated in one or more purification steps to improve one or more properties. Many such purification techniques, such as distillation, solvent extraction, acid or base extraction, filtration and percolation are known to those skilled in the art. Rerefined oils are obtained by processes similar to those used to obtain refined oils applied to refined oils which have been already used in service. Such rerefined oils are also known as reclaimed or reprocessed oils and often are additionally processed by techniques for removal of spent additives and oil breakdown products.

Generally the lubricants of the present invention contain an amount of the composition of this invention sufficient to provide it with friction modification and/or deposit softening properties. Normally, this amount employed will be about 0.05 percent to about 20 percent, preferably about 0.1 percent to about 10 percent of the total weight of the lubricating composition. This amount is exclusive of solvent/diluent medium. In lubricating compositions operated under extremely adverse conditions, such as lubricating compositions for marine diesel engines, the compositions of this invention may be present in amounts of up to about 30 percent by weight, or more, of the total weight of the lubricating composition.

The term "minor amount" as used in the specification and appended claims is intended to mean that when a composition contains a "minor amount" of a specific material that amount is less than 50 percent by weight of the composition.

The term "major amount" as used in the specification and appended claims is intended to mean that when a composition contains a "major amount" of a specific material that amount is more than 50 percent by weight of the composition.

The invention also contemplates the use of other additives in combination with the compositions of this invention. Such additives include, for example, detergents and dispersants of the ash-producing or ashless type, corrosion- and oxidation-inhibiting agents, pour point depressing agents, extreme pressure agents, antiwear agents, color stabilizers and anti-foam agents.

The ash-producing detergents are exemplified by oil-soluble neutral and basic salts of alkali or alkaline earth metals with sulfonic acids, carboxylic acids, or organic phosphorus acids characterized by at least one direct carbon-to-phosphorus linkage such as those prepared by the treatment of an olefin polymer (e.g., polyisobutene having a molecular weight of 1000) with a phosphorizing agent such as phosphorus trichloride, phosphorus heptasulfide, phosphorus pentasulfide, phosphorus trichloride and sulfur, white phosphorus and a sulfur halide, or phosphorothioic chloride. The most commonly used salts of such acids are those of sodium, potassium, lithium, calcium, magnesium, strontium and barium.

The term "basic salt" is used to designate metal salts wherein the metal is present in stoichiometrically larger amounts than the organic acid radical. The commonly employed methods for preparing the basic salts involve heating a mineral oil solution of an acid with a stoichiometric excess of a metal neutralizing agent such as the metal oxide, hydroxide, carbonate, bicarbonate, or sulfide at a temperature of at least about 50°C. and filtering the resulting mass. The use of a "promoter" in the neutralization step to aid the incorporation of a large excess of metal likewise is known. Examples of compound useful as the promoter include phenolic substances such as phenol, naphthol, alkylphenol, thiophenol, sulfurized alkylphenol, and condensation products of formaldehyde with a phenolic substance; alcohols such as methanol, 2-propanol, octyl alcohol, cellosolve, carbitol, ethylene glycol, stearyl alcohol, and cyclohexyl alcohol; and amines such as aniline, phenylenediamine, phenothiazine, phenyl-naphthylamine, and dodecylamine. A particularly effective method for preparing the basic salts comprises mixing an acid with an excess of a basic alkaline earth metal neutralizing agent and at least one alcohol promoter, and carbonating the mixture at an elevated temperature such as 60-200°C.

Ashless detergents and dispersants are so called despite the fact that, depending on its constitution, the dispersant may upon combustion yield a nonvolatile material such as boric oxide or phosphorus pentoxide; however, it does not ordinarily contain metal and therefore does not yield a metal-containing ash on combustion. Many types are known in the art, and any of them are suitable for use in the lubricant compositions of this invention. The following are illustrative:
(1) Reaction products of carboxylic acids (or derivatives thereof) containing at least about 34 and preferably at least about 54 carbon atoms with nitrogen-containing compounds such as amine, organic hydroxy compounds such as phenols and alcohols, and/or basic inorganic materials. Examples of these "carboxylic dispersants" are described in British Patent 1,306,529 and in many U.S. patents including the following:

| | | |
|---|---|---|
| 3,163,603 | 3,351,552 | 3,541,012 |
| 3,184,474 | 3,381,022 | 3,543,678 |
| 3,215,707 | 3,399,141 | 3,542,680 |
| 3,219,666 | 3,415,750 | 3,567,637 |
| 3,271,310 | 3,433,744 | 3,574,101 |
| 3,272,746 | 3,444,170 | 3,576,743 |
| 3,281,357 | 3,448,048 | 3,630,904 |
| 3,306,908 | 3,448,049 | 3,632,510 |
| 3,311,558 | 3,451,933 | 3,632,511 |
| 3,316,177 | 3,454,607 | 3,697,428 |
| 3,340,281 | 3,467,668 | 3,725,441 |
| 3,341,542 | 3,501,405 | Re 26,433 |
| 3,346,493 | 3,522,197 | |

(2) Reaction products of relatively high molecular weight aliphatic or alicyclic halides with amines, preferably polyalkylene polyamines. These may be characterized as "amine dispersants" and examples thereof are described for example, in the following U.S. patents:

| | |
|---|---|
| 3,275,554 | 3,454,555 |
| 3,438,757 | 3,565,804 |

(3) Reaction products of alkyl phenols in which the alkyl group contains at least about 30 carbon atoms with aldehydes (especially formaldehyde) and amines (especially polyalkylene polyamines), which may be characterized as "Mannich dispersants". The materials described in the following U.S. patents are illustrative:

| | | |
|---|---|---|
| 2,459,112 | 3,442,808 | 3,591,598 |
| 2,962,442 | 3,448,047 | 3,600,372 |
| 2,984,550 | 3,454,497 | 3,634,515 |
| 3,036,003 | 3,459,661 | 3,649,229 |
| 3,166,516 | 3,461,172 | 3,697,574 |
| 3,236,770 | 3,493,520 | 3,725,277 |
| 3,355,270 | 3,539,633 | 3,725,480 |
| 3,368,972 | 3,558,743 | 3,726,882 |
| 3,413,347 | 3,586,629 | 3,980,569 |

(4) Products obtained by post-treating the carboxylic, amine or Mannich dispersants with such reagents as urea, thiourea, carbon disulfide, aldehydes, ketones, carboxylic acids, hydrocarbon-substituted succinic anhydrides, nitriles, epoxides, boron compounds, phosphorus compounds or the like. Exemplary materials of this kind are described in the following U.S. patents:

| | | | |
|---|---|---|---|
| 3,036,003 | 3,282,955 | 3,493,520 | 3,639,242 |
| 3,087,936 | 3,312,619 | 3,502,677 | 3,649,229 |
| 3,200,107 | 3,366,569 | 3,513,093 | 3,649,659 |
| 3,216,936 | 3,367,943 | 3,533,945 | 3,658,836 |
| 3,254,025 | 3,373,111 | 3,539,633 | 3,697,574 |
| 3,256,185 | 3,403,102 | 3,573,010 | 3,702,757 |
| 3,278,550 | 3,442,808 | 3,579,450 | 3,703,536 |
| 3,280,234 | 3,455,831 | 3,591,598 | 3,704,308 |
| 3,281,428 | 3,455,832 | 3,600,372 | 3,708,422 |

(5) Interpolymers of oil-solubilizing monomers such as decyl methacrylate, vinyl decyl ether and high molecular weight olefins with monomers containing polar substituents, e.g., aminoalkyl acrylates or acrylamides and poly-(oxyethylene)-subsituted acrylates. These may be characterized as "polymeric dispersants" and examples thereof are disclosed in the following U.S. patents:

| | |
|---|---|
| 3,329,658 | 3,666,730 |
| 3,449,250 | 3,687,849 |
| 3,519,565 | 3,702,300 |

Extreme pressure agents and corrosion- and oxidation-inhibiting agents are exemplified by chlorinated aliphatic hydrocarbons such as chlorinated wax; organic sulfides and polysulfides such as benzyl disulfide, bis(chlorobenzyl)disulfide, dibutyl tetrasulfide, sulfurized methyl ester of oleic acid, sulfurized alkylphenol, sulfurized dipentene, and sulfurized terpene; phosphosulfurized hydrocarbons such as the reaction product of a phosphorus sulfide with turpentine or methyl oleate, phosphorus esters including principally dihydrocarbon and trihydrocarbon phosphites such as dibutyl phosphite, diheptyl phosphite, dicyclohexyl phosphite, pentylphenyl phosphite, dipentylphenyl phosphite, tridecyl phosphite, distearyl phosphite, dimethyl napthyl phosphite, oleyl 4-pentylphenyl phosphite, polypropylene (molecular weight 500)-substituted phenyl phosphite, diisobutyl-substituted phenyl phosphite; metal thiocarbamates, such as zinc dioctyldithiocarbamate, and barium heptylphenyl dithiocarbamate; Group II metal phosphorodithioates such as zinc dicyclohexylphosphorodithioate, zinc dioctylphosphorodithioate, barium di(heptylphenyl)-phosphorodithioate, cadmium dinonylphosphorodithioate, and the zinc salt of a phosphorodithioic acid produced by the reaction of phosphorus pentasulfide with an equimolar mixture of isopropyl alcohol and n-hexyl alcohol.

The compositions of this invention can be added directly to the lubricant. Preferably, however, they are diluted with a substantially inert, normally liquid organic diluent such as mineral oil, naphtha, benzene, toluene or xylene, to form an additive concentrate. These concentrates usually contain from about 10 percent to 90 percent by weight of the composition of this invention and may contain, in addition, one or more other additives known in the art or described hereinabove.

The fuel compositions of the present invention contain a major proportion of a normally liquid fuel, usually a hydrocarbonaceous petroleum distillate fuel such as motor gasoline as defined by ASTM Specification D-439-73 and diesel fuel or fuel oil as defined by ASTM Specification D-396. Normally liquid fuel compositons comprising nonhydrocarbonaceious materials such as alcohols, ethers, organonitro compounds and the like (e.g., methanol, ethanol, diethyl ether, methyl ethyl ether, nitromethane) are also within the scope of this invention as are liquid fuels derived from vegetable or mineral sources such as corn, alfalfa, shale and coal. Normally liquid fuels which are mixtures of one or more hydrocarbonaceous fuels and one or more nonhydrocarbonaceous materials are also contemplated. Examples of such mixtures are combinations of gasoline and ethanol, and diesel fuel and ether. Particularly preferred is gasoline, that is, a mixture of hydrocarbons having an ASTM boiling point of about 60°C at the 10 percent distillation point to about 205°C at the 90 percent distillation point.

Generally, these fuel compositions contain an amount of the composition of this invention sufficient to impart friction modification and/or deposit softening properties to the fuel; usually this amount is bout 0.001 to about 5 percent (based on the weight of the final composition), preferably 0.001 percent to 1.0 percent.

The fuel compositions of this invention can contain, in addition to the compositions of this invention, other additives which are well known to those of skill in the art. These can include antiknock agents such as tetraalkyl lead compounds, lead scavengers such as halo-alkanes (e.g., ethylene dichloride and ethylene dibromide), deposit preventors or modifiers such as triaryl phosphates, dyes, cetane improvers, auxiliary antioxidants such as 2,6-ditertiary-butyl-4-methylphenol, rust inhibitors such as alkylated succinic acids and anhydrides, bacteriostatic agents, gum inhibitors, metal deactivators, demulsifiers, upper cylinder lubricants, anti-icing agents and the like.

In certain preferred fuel compositions of the present invention, the aforedescribed compositions are combined with an ashless dispersant in gasoline. Such ashless dispersants are preferably esters of a mono- or polyol and a high molecular weight mono- or polycarboxylic acid acylating agent containing at least 30 carbon atoms in the acyl moiety. Such esters are well known to those of skill in the art. See, for example, French Patent No. 1,396,645, British Patent Nos. 981,850 and 1,055,337 and U.S. Patent Nos. 3,255,108; 3,311,558; 3,331,776; 3,346,354; 3,522,179; 3,579,450; 3,542,680; 3,381,022; 3,639,242; 3,697,428; 3,708,522; and British Patent Specification 1,306,529.

Generally, the weight ratio of the compositions of this invention to the aforesaid ashless dispersants is about 0.1 to about 10.0, preferably about 1 to about 10 parts of composition to 1 part ashless dispersant. In still another embodiment of this invention, the inventive additives are combined with Mannich condensation products formed from substituted phenols, aldehydes, polyamines, and substituted pyridines. Such condensation products are described in U.S. Patent Nos. 3,649,659; 3,558,743; 3,539,633; 3,704,308; and 3,725,277.

The compositions of this invention can be added directly to the fuel to form the fuel compositions of this invention or they can be diluted with a substantially inert, normally liquid organic solvent/diluent such as mineral oil, xylene, or a normally liquid fuel as described above, to form an additive concentrate which is then added to the fuel in sufficient amounts to form the inventive fuel composition described herein. These concentrates generally contain about 10 to 90 percent of the compositions of this invention and can contain in addition any of the abovedescribed conventional additives, particularly the aforedescribed ashless dispersants in the aforesaid proportions. The remainder of the concentrate is the solvent/diluent.

The lubricant fuel and additive concentrate compositions of this invention are exemplified by the following Table. All amounts other than those for mineral oil are exclusive of oil used as diluent.

| PARTS BY WEIGHT | |
|---|---|
| COMPONENT | I |
| Mineral Oil | 92.87 |
| Polybutenyl succinic anhydride ethylene polyamine reaction product | 1.94 |
| Styrene-alkyl maleate copolymer | 1.79 |
| Zinc isooctylphosphrodithioate | .78 |
| Zinc methylamylphosphoridithioate | .67 |

| | IV |
|---|---|
| Basic calcium petroleum sulfonate | .52 |
| Basic sodium petroleum sulfonate | .42 |
| Silicon anti-foamant | .005 |
| Product of Example | 1.000 |

The fuel consumption of engines lubricated with compositions of this invention is measurably lower than that of engines lubricated with previously known lubricants. This can be shown by the Motored Engine Friction Horsepower Test, in which an engine is driven by a dynamometer at constant temperature as engine rpm. and torque are measured by a digital tachometer and a precision dial manometer, respectively. Friction horsepower, as calculated from these values, is roughly proportional to fuel consumed and thus decreases with improved fuel economy.

## Claims

1. A substituted succinimide derivative represented by the general formula: 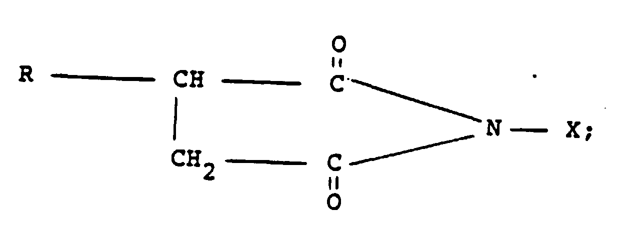 wherein X is 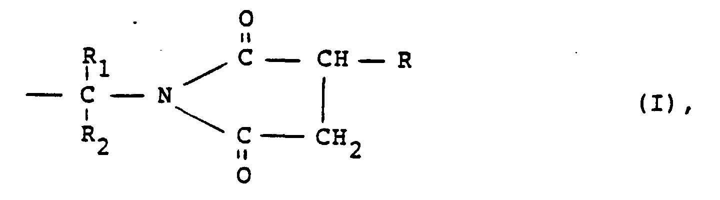 wherein R is a hydrocarbon-based group containing from about 8 up to about 35 carbon atoms, and R₁ and R₂ are each independently hydrogen or an alkyl group containing up to 8 carbon atoms.

2. A substituted succinimide derivative according to Claim 1 wherein R is an aliphatic hydrocarbon group, free from acetylenic unsaturation, containing from about 10 up to about 30 carbon atoms, in which at least 8 carbon atoms are in a straight-chain configuration and at least one of R₁ and R₂ is hydrogen.

3. A process for the preparation of a substituted succinimide derivative according to Claim 1 which comprises reacting in the presence of a catalytic amount of base:
A) one or more substituted succinimides represented by the formula: 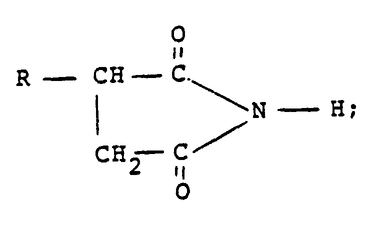 wherein R is a hydrocarbon-based group containing from about 8 up to about 35 carbon atoms; with
B) one or more carbonyl compounds represented by the formula: 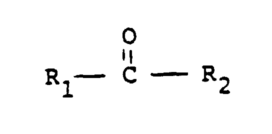 wherein R₁ and R₂ are each independently hydrogen or an alkyl radical containing up to 8 carbon atoms.

4. A process according to Claim 3 wherein at least one of R₁ and R₂ is hydrogen.

5. A process according to Claim 4 wherein R₁ and R₂ are hydrogen and about one mole of water for each mole of carbonyl compound is removed from the reaction and wherein there is at least about one mole of carbonyl compound for every two moles of succinimide.

6. An additive concentrate comprising a substantially inert, normally liquid organic diluent and from about 10 percent up to about 90 percent by weight of a substituted succinimide derivative according to Claim 1 or 2, or prepared according to any of claims 3 to 5.

7. A lubricating composition comprising a major amount of a lubricating oil and a minor amount of at least one substituted succinimide derivative according to Claim 1 or 2, or prepared according to any of claims 3 to 5.

8. A fuel composition comprising a major amount of a normally liquid fuel and a minor amount of at least one substituted succinimide derivative according to Claim 1 or 2, or prepared according to any of claims 3 to 5.

9. A method for reducing fuel consumption in an internal combustion engine which comprises lubricating said engine during operation with at least one lubricating substituted succinimide derivative according to Claim 1 or 2, or prepared according to any of claims 3 to 5.

## Patentansprüche

1. Substituiertes Succinimidderivat der allgemeinen Formel 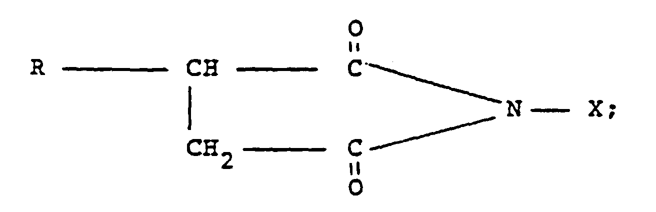 in der der Rest X die Gruppe 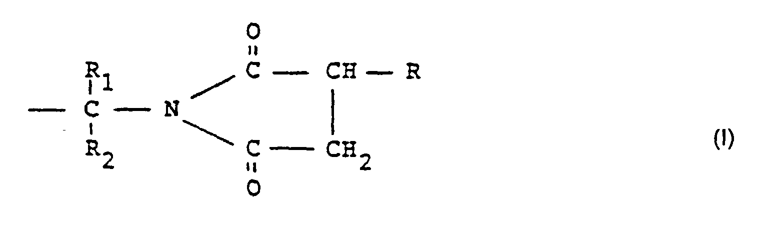 ist, in der der Rest R eine Gruppe auf Kohlenwasserstoffbasis mit etwa 8 bis etwa 35 Kohlenstoffatomen ist und die Reste R₁ und R₂ jeweils unabhängig Wasserstoffatome oder Alkylgruppen mit bis zu 8 Kohlenstoffatomen sind.

2. Substituiertes Succinimidderivat gemäß Anspruch 1, wobei der Rest R eine aliphatische Kohlenwasserstoffgruppe ist, die frei von acetylenischer Ungesättigtheit ist und etwa 10 bis etwa 30 Kohlenstoffatome enthält, wobei wenigstens 8 Kohlenstoffatome in einer geradkettigen Konfiguration vorliegen und wenigstens einer der Reste R₁ und R₂ ein Wasserstoffatom ist.

3. Verfahren zur Herstellung eines substituierten Succinimidderivats gemäß Anspruch 1, umfassend Umsetzen in Gegenwart einer katalytischen Menge von Base:
A) eines oder mehrerer substituierter Succinimide der allgemeinen Formel 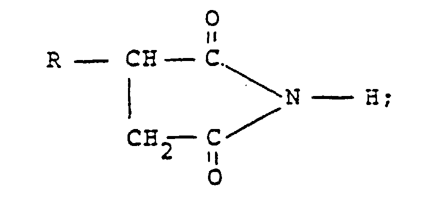 in der der Rest R eine Gruppe auf Kohlenwasserstoffbasis mit etwa 8 bis etwa 35 Kohlen
stoffatomen ist; mit
B) einer oder mehreren Carbonylverbindungen der allgemeinen Formel 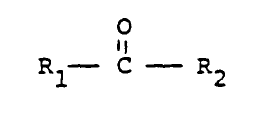 in der die Reste R₁ und R₂ jeweils unabhängig Wasserstoffatome oder Alkylgruppen mit bis zu 8 Kohlenstoffatomen sind.

4. Verfahren gemäß Anspruch 3, wobei wenigstens einer der Reste R₁ und R₂ ein Wasserstoffatom ist.

5. Verfahren gemäß Anspruch 4, wobei die Reste R₁ und R₂ Wasserstoffatome sind und wobei etwa 1 Mol Wasser pro Mol Carbonylverbindung aus dem Reaktionsgemisch entfernt wird und wobei wenigstens etwa 1 Mol Carbonylverbindung für jeweils 2 Mol Succinimid vorliegt.

6. Additivkonzentrat, das ein im wesentlichen inertes, normalerweise flüssiges organisches Verdünnungsmittel und etwa 10 bis etwa 90 Gew.-% eines substituierten Succinimidderivats gemäß Anspruch 1 oder 2 oder hergestellt gemäß einem der Ansprüche 3 bis 5 umfaßt.

7. Schmiermittelzusammensetzung, die eine Hauptmenge eines Schmieröls und eine geringere Menge von wenigstens einem substituierten Succinimidderivat gemäß Anspruch 1 oder 2 oder hergestellt gemäß einem der Ansprüche 3 bis 5 umfaßt.

8. Treib- bzw. Brennstoffzusammensetzung, die eine Hauptmenge eines normalerweise flüssigen Treib- bzw. Brennstoffs und eine geringere Menge von wenigstens einem substituierten Succinimidderivat gemäß Anspruch 1 oder 2 oder hergestellt gemäß einem der Ansprüche 3 bis 5 umfaßt.

9. Verfahren zur Verringerung des Treib- bzw. Brennstoffverbrauchs eines Motors mit innerer Verbrennung, das das Schmieren des Motors beim Betrieb mit wenigstens einem schmierenden substituierten Succinimidderivat gemäß Anspruch 1 oder 2 oder hergestellt gemäß einem der Ansprüche 3 bis 5 umfaßt.

## Revendications

1. Un dérivé de succinimide substitué, représenté par la formule générale : 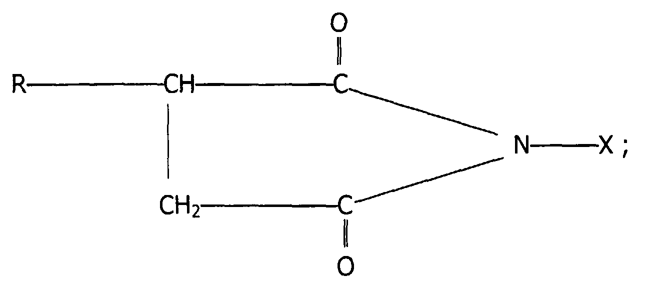 dans laquelle Y est 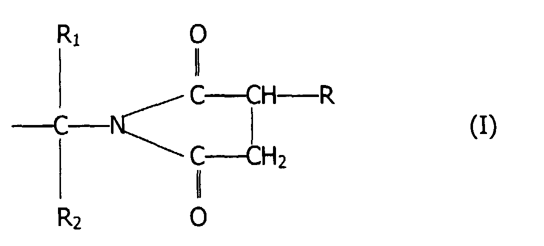 dans laquelle R est un groupe à base d'hydrocarbure, qui contient d'environ 8 à environ 35 atomes de carbone, et R₁ et R₂ sont chacun indépendamment de l'hydrogène ou un groupe alkyle renfermant jusqu'à 8 atomes de carbone.

2. Un dérivé de succinimide substitué selon Ia revendication 1, dans lequel R est un groupe hydrocarbure aliphatique, exempt d'insaturation acétylénique, qui contient d'environ 10 à environ 30 atomes de carbone, dans lequel au moins 8 atomes de carbone sont en configuration à chaîne linéaire et au moins un des R₁ et R₂ est de l'hydrogène.

3. Un procédé pour la préparation d'un dérivé de succinimide substitué selon la revendication 1, qui consiste à faire réagir, en présence d'une quantité catalytique d'une base :
(A) un ou plus d'un succinimide substitué, représenté par la formule : 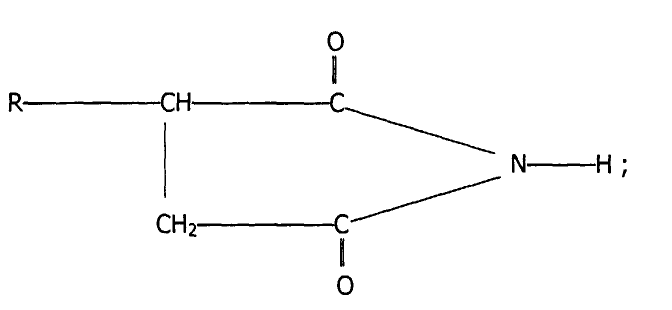 dans laquelle R est un groupe à base d'hydrocarbure, qui contient d'environ 8 à environ 35 atomes de carbone, avec
(B) un ou plus d'un composé carbonyle représenté par la formule : 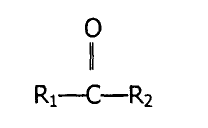 dans laquelle R₁ et R₂ représentent chacun indépendamment de l'hydrogène ou un radical alkyle renfermant jusqu'à 8 atomes de carbone.

4. Un procédé selon la revendication 3, dans lequel au moins un des R₁ et R₂ est de l'hydrogène.

5. Un procédé selon la revendication 4, dans lequel R₁ et R₂ sont de l'hydrogène et environ 1 mole d'eau pour chaque mole de composé carbonyle est éliminée de la réaction et dans lequel il existe au moins environ une mole de composé carbonyle pour chaque deux moles de succinimide.

6. Un concentré additif, comportant un diluant organique essentiellement inerte, liquide sous les conditions normales, et d'environ 10 % à environ 90 % en poids d'un dérivé succinimide substitué selon la revendication 1 ou 2, ou bien préparé selon l'une quelconque des revendications 3 à 5.

7. Une composition lubrifiante comportant une quantité prépondérante d'une huile lubrifiante et une quantité plus faible d'au moins un dérivé de succinimide substitué selon la revendication 1 ou 2, ou bien préparé selon l'une quelconque des revendications 3 à 5.

8. Une composition de carburant comportant une quantité prépondérante d'un carburant liquide sous les conditions normales et une quantité plus faible d'au moins un dérivé de succinimide substitué selon la revendication 1 ou 2, ou bien préparé selon l'une quelconque des revendications 3 à 5.

9. Un procédé pour réduire la consommation de carburant dans un moteur à combustion interne, qui comprend la lubrification dudit moteur pendant le fonctionnement avec au moins un dérivé lubrifiant de succinimide substitué selon la revendication 1 ou 2, ou bien préparé selon l'une quelconque des revendications 3 à 5.
